Europäisches Patentamt

European Patent Office

Office européen des brevets

(11) Veröffentlichungsnummer: **0 342 398 B1**

(12) **EUROPÄISCHE PATENTSCHRIFT**

(45) Veröffentlichungstag der Patentschrift: **16.12.92**

(21) Anmeldenummer: **89107507.9**

(22) Anmeldetag: **26.04.89**

(51) Int. Cl.⁵: **G01N 33/53**, G01N 33/80, G01N 33/96

(54) Verfahren zum Nachweis von Antikörpern gegen erythrozytenspezifische Antigene.

(30) Priorität: **14.05.88 DE 3816569**

(43) Veröffentlichungstag der Anmeldung:
**23.11.89 Patentblatt 89/47**

(45) Bekanntmachung des Hinweises auf die
Patenterteilung:
**16.12.92 Patentblatt 92/51**

(84) Benannte Vertragsstaaten:
**DE FR GB IT**

(56) Entgegenhaltungen:
**DE-C- 2 008 493**
**US-A- 3 406 121**

(73) Patentinhaber: **Giannitsis, Dimitrios, Dr.**
**Stabelstrasse 6**
**W-7500 Karlsruhe(DE)**

(72) Erfinder: **Giannitsis, Dimitrios, Dr.**
**Stabelstrasse 6**
**W-7500 Karlsruhe(DE)**

(74) Vertreter: **Lichti, Heiner, Dipl.-Ing. et al**
**Patentanwälte Dr. Ing. Hans Lichti Dipl.-Ing.**
**Heiner Lichti Dipl.-Phys. Dr.rer.nat. Jost Lem-**
**pert Postfach 410760**
**W-7500 Karlsruhe 41(DE)**

EP 0 342 398 B1

## Beschreibung

Die Erfindung betrifft ein Verfahren zum Nachweis von in Blutserum enthaltenen Antikörpern gegen Erythrozytenspezifische Antigene.

Bei der Bluttransfusion können eine Reihe von Störungen auftreten. Neben den posttransfusionellen Infektionen, kreislaufbelastenden volämischen Hypertransfusionen, Eiweißunverträglichkeiten und den allergischen Transfusionsreaktionen stellen serologisch bedingte Transfusionsstörungen eine schwerwiegende Komplikation bei der Bluttransfusion dar. Die Häufigkeit hämolytischer Transfusionszwischenfälle wird mit 1:5000 angegeben, wobei über eine Letalität von 10 % berichtet wird (Spielmann/Seidl, "Einführung in die Immunhämatologie und Transfusionskunde" 2. Auflage, 1982, Verlag Chemie; P.L. Mollison, "Blood Transfusion in Clinical Medicine", 7. Auflage, 1983, Blackwell Scientific Publications). Für polytransfundierte Patienten liegt das Risiko einer Komplikation noch erheblich höher.

Um solche Transfusionsstörungen zu vermeiden, ist eine serologische Verträglichkeitsuntersuchung vor jeder Transfusion geboten ("Richtlinien zur Blutgruppenbestimmung und Bluttransfusion" Herausgeber: Wissenschaftlicher Beirat der Bundesärztekammer und des Bundesgesundheitsamtes, Neufassung 1987, Deutscher-Ärzte-Verlag). Diese Verträglichkeitsprobe ist praktisch ein indirekter Coombstest zwischen Spendererythrozyten und Empfängerplasma.

In vielen Fällen muß eine solche Verträglichkeitsuntersuchung in kurzer Frist durchführbar sein, was inbesondere beim Nachweis erythrozytärer Antikörper Schwierigkeiten bereitet. Ein bekannter Schnelltest (Lalezari) (Sandra S. Ellisor und Margaret E. Wallace "Blood Bank Reagents: What to use and when" AABB 1985, S. 32-35), der sogenannte Polybren-Test, basiert auf dem Prinzip, daß Polykationen, wie z. B. Polybren, Protamin, Polylysin etc. durch ihre positiv geladenen Makromoleküle die negative Ladung der Erythrozyten so stark reduzieren, daß es zu einer unspezifischen Hämagglutination kommt. Diese Agglutination ist jedoch durch hypertone Salzlösungen wieder auflösbar, also wenig stabil. Der Polybren-Test läuft im wesentlichen in drei Schritten ab. Im ersten Schritt sorgt eine LISS-Lösung (Low Ionic Medium) für eine verstärkte Antikörperaufladung der Erythrozyten. Im zweiten Schritt wird das Zetapotential durch Einbringung quartärer Aminogruppen (Amonium-Polymere) so stark erniedrigt, daß es zu einer unspezifischen Aggregation der Erythrozyten kommt. Im dritten Schritt wird durch einen Wechsel des Reaktionsmilieus mit einer hypertonen Salzlösung, z. B. Natrium-Citrat, der überwiegende Teil unspezifischer Bindungen aufgelöst, bevor auch die prinzipiell reversiblen Antikörper-bedingten Verbindungen angegriffen werden.

Der Vorteil der Methode besteht vor allem darin, daß der Test innerhalb von 4 bis 5 Minuten zur Nachweisreaktion führt und ferner die Durchführung des Testes vergleichsweise einfach ist. Von Nachteil hingegen ist die Tatsache, daß eine positive Reaktion nicht einfach abzulesen und infolgedessen eine entsprechende Erfahrung erforderlich ist. Ferner ist nachteilig, daß die Agglutination nur einige wenige Minuten bestehen bleibt und sich dann wieder auflöst. Schließlich erfaßt diese Nachweismethode nicht alle blutgruppenspezifischen Antikörper, z. B. nicht Anti-Kell.

Der Erfindung liegt die Aufgabe zugrunde, eine schnelle und einfache Testmethode vorzuschlagen, die zu einem stabilen Agglutinat führt und alle blutgruppenspezifischen Antikörper erfaßt.

Diese Aufgabe wird erfindungsgemäß dadurch gelöst, daß eine Serumverdünnung mit einer Erythrozyten-Suspension in 0,9 % NaCl-Lösung als Antigenträger angesetzt und mit einem Low Ionic Medium (LISS = Low Ionic Strength Solution) gemischt, die Mischung inkubiert, daraufhin eine Aggregationslösung in Form von säurelöslichen Proteinen aus menschlichen Leukozytenkernen zugegeben, die Mischung erneut inkubiert und anschließend zentrifugiert, der Überstand abgetrennt und die verbleibende Mischung mit Heparin-Natrium oder einer organischen Säure neutralisiert und auf Agglutination geprüft wird.

Beim erfindungsgemäßen Verfahren werden säurelösliche Proteine (SLP) aus menschlichen Leukozytenkernen als Aggregationsmittel anstelle von Polybren oder anderen Polykationen, z. B. Protamin, Polylysin etc., eingesetzt und hierdurch die Test-Erythrozyten aggregiert. Nach kurzer Inkubationszeit wird Antikörper-haltiges Serum oder Plasma zugesetzt, wiederum kurz inkubiert und dann zentrifugiert. Der Überstand wird abgegossen und nach Zugabe des Neutralisationsmediums (Heparin-Natrium oder organische Säuren) die sedimentierten Erythrozyten aufgeschüttelt. Bei negativen Reaktionen werden die Erythrozyten aufgelöst, bei positiven Reaktionen bleiben die Erythrozyten agglutiniert. Das Agglutinat ist über Stunden hinweg ablesbar. Diese Nachweismethode ist gegenüber bisherigen Verfahren empfindlicher, schneller und einfach durchführbar. Ferner läßt sich diese Methode für folgende Anwendungen einsetzen: Nachweis von Antikörpern, anstelle des indirekten Coombstestes, Schnelldifferenzierung von Antikörpern sowie Verträglichkeitsuntersuchungen zwischen Spender- und Empfängerblut bei dringenden, notfallmäßigen Bluttransfusionen.

Die Erfindung setzt säurelösliche Proteine (SLP) aus menschlichen Leukozyten-Zellkernen als Aggregationsmittel anstelle von Polybren oder anderen Polykationen, z. B. Protamin, Polylysin etc., ein. Der Vorteil dieser säurelöslichen Proteine im Vergleich zu Polybren besteht darin, daß sie eine Gruppe von fünf verschiedenen Protein-Fraktionen umfassen, während Polybren lediglich ein synthetisches Polypeptid ist. Die erfindungsgemäß eingesetzte Gruppe von Proteinen (D. Giannitsis "Histone der Zellkerne menschlicher Leukozyten" Habilitationsschrift 1985, Medizinische Fakultät der Universität des Saarlandes, Saarbrücken) besteht aus verschiedenen positiv geladenen Makromolekülen, die die negative Ladung der Erythrozyten unterschiedlich reduzieren können. Daraus resultieren unspezifische Hämagglutinationen verschiedener Stärken in Abhängigkeit von der verwendeten SLP-Fraktion. Diese Hämagglutination ist zwar auflösbar, jedoch im Gegensatz zur Polybren-Technik nicht durch Zusatz hypertoner Salzlösungen, sondern durch Neutralisation der SLP mit Heparin-Natrium oder organischen Säuren, z. B. Milchsäure, Zitronensäure etc. Die Auswahl der verwendeten Säure bestimmt in Abhängigkeit von der Anzahl der freien Carboxylgruppen im wesentlichen nur den Neutralisationseffekt. Die Hämagglutination bleibt jedoch trotz Neutralisation der SLP mit Heparin oder organischer Säure bei Verbindungen, die durch Blutgruppenantikörper bedingt sind, irreversibel. Diese irreversible Agglutination bleibt über Stunden ablesbar. Ein weiterer Vorteil der erfindungsgemäßen Methode liegt darin, daß aufgrund der hohen Nachweisempfindlichkeit alle Blutgruppenantikörper auch in niedrigen Konzentrationen festgestellt werden können und mit dem indirekten Coombstest unmittelbar vergleichbar sind.

Bei dem erfindungsgemäßen Verfahren wird als Antigenträger vorzugsweise eine 2 bis 5 %ige Erythrozyten-Suspension in 0,9 % NaCl-Lösung verwendet.

Für die Aggregationslösung empfiehlt sich eine Stammlösung aus 1 g säurelöslichen Proteinen aus menschlichen Leukozytenkernen in 100 ml Aqua dest., die im Verhältnis 1:10 mit 0,9 % NaCl zu einer Gebrauchslösung verdünnt wird.

Die Neutralisationslösung kann aus einer Stammlösung aus 10 g organischer Säure und 100 ml Aqua dest. durch Verdünnen im Verhältnis 1:10 mit 0,9 % NaCl gewonnen werden.

Als organische Säuren können Zitronensäure, Milchsäure oder artverwandte Säuren verwendet werden.

Für die Neutralisationslösung kann Heparin-Natrium pH 5, 5000 IU/ml (z. B. der Firma Ratiopharm) verwendet werden.

Die Inkubierung nach Zugabe von LISS, wie auch die Inkubierung nach Zugabe der säurelöslichen Proteine kann bei Raumtemperatur in einer Zeit bis maximal 2 Minuten erfolgen.

Das Zentrifugieren nach Zugabe der Aggregationslösung und Inkubieren der Mischung erfolgt mit Vorteil bei ca. 1000 l/min. Ferner ist von Vorteil, wenn die Mischung auch nach Zugabe der Neutralisationslösung anzentrifugiert wird, beispielsweise bei ca. 1000 l/min. Nach jedem Zentrifugieren empfiehlt sich ein Aufschütteln der Mischung, da die Erythrozyten sedimentieren.

Es empfiehlt sich, neben der Testreihe eine Kontrollreihe durchzuführen. Der Kontrollnachweis erfolgt statt mit saurelöslichen Proteinen aus menschlichen Leukozytenkernen durch eine Protaminlösung, z. B. Protamin-HCl. Eine verwertbare Protaminlösung wird beispielsweise aus einer Stammlösung mit 1 g Protamin-HCl 100 ml Aqua dest. durch Verdünnen im Verhältnis 1:10 mit 0,9 % NaCl erhalten.

Die Empfindlichkeit des Tests wird in Verdünnungsreihen des Anti-Serums untersucht. Hierzu wird eine Verdünnung des Anti-Serums mit 0,9 % NaCl-Lösung hergestellt. Wichtig ist ferner das Mitführen einer negativen Kontrolle. Nachfolgend sind die Schritte im einzelnen angegeben:

1. Röhrchen für den Test und die negative Kontrolle beschriften.

2. 100 $\mu$l Serumverdünnung in jedes Röhrchen vorlegen.

3. 50 $\mu$l 2 bis 5 %ige Erythrozyten-Suspension in die entsprechenden Röhrchen geben.

4. 100 $\mu$l Low Ionic Medium in jedes Röhrchen geben und gut mischen.

5. Ansätze 1 Minute bei Raumtemperatur inkubieren.

6. 100 $\mu$l Aggregationslösung (säurelösliche Proteinen menschlichen Leukozytenkernen bzw. Protamin-HCl) in die Röhrchen geben und mischen.

7. Ansätze 15 Sekunden bei Raumtemperatur inkubieren.

8. Röhrchen 1 Minute bei 1000 l/min zentrifugieren und den Überstand vollständig abgießen.

9. Die Röhrchen in einen Ständer stellen und die am Boden abgesetzten Erythrozyten leicht aufschütteln.

10. 100 $\mu$l Neutralisationsmedium in jedes Röhrchen geben und mischen.

11. Die Röhrchen bei 1000 l/min anzentrifugieren, leicht aufschütteln, abstellen und auf Agglutination prüfen. Bei dem Testsubstrat kann die Ablesung innerhalb 1 Minute nach Zugabe von Neutralisationsmedium erfolgen; das Ergebnis bleibt für Stunden unverändert.

**Patentansprüche**

1. Verfahren zum Nachweis von in Blutserum enthaltenen Antikörpern gegen Erythrozyten-spezifische Antigene, indem eine Serumverdünnung mit einer Erythrozyten-Suspension in 0,9 % NaCl-Lösung als Antigenträger angesetzt und mit einen Low Ionic Medium (LISS = Low Ionic Strength Solution) gemischt, die Mischung inkubiert, daraufhin eine Aggregationslösung in Form von säurelöslichen Proteinen aus menschlichen Leukozytenkernen zugegeben, die Mischung erneut inkubiert und anschließend zentrifugiert, der Überstand abgetrennt und die verbleibende Mischung mit Heparin-Natrium oder einer organischen Säure neutralisiert und auf Agglutination geprüft wird.

2. Verfahren nach Anspruch 1, dadurch gekennzeichnet, daß als Antigenträger eine 2 bis 5 %ige Erythrozyten-Suspension in 0,9 % NaCl-Lösung verwendet wird

3. Verfahren nach Anspruch 1 oder 2, dadurch gekennzeichnet, daß für die Aggregationslösung eine Stammlösung aus 1 g säurelöslichen Proteinen aus menschlichen Leukozytenkernen in 100 ml Aqua dest. hergestellt und diese im Verhältnis 1:10 mit 0,9 % NaCl zu einer Gebrauchslösung verdünnt wird.

4. Verfahren nach einem der Ansprüche 1 bis 3, dadurch gekennzeichnet, daß für die Neutralisationslösung eine Stammlösung aus 10 g organischer Säure und 100 ml Aqua dest. hergestellt und diese im Verhältnis 1:10 mit 0,9 % NaCl zu einer Gebrauchslösung verdünnt wird.

5. Verfahren nach Anspruch 4, dadurch gekennzeichnet, daß als organische Säure Zitronensäure oder Milchsäure verwendet wird.

6. Verfahren nach einem der Ansprüche 1 bis 3, dadurch gekennzeichnet, daß für die Neutralisationslösung Heparin-Natrium pH 5, 5000 IU/ml verwendet wird.

7. Verfahren nach einem der Ansprüche 1 bis 6, dadurch gekennzeichnet, daß die Mischung bei Raumtemperatur inkubiert wird.

8. Verfahren nach einem der Ansprüche 1 bis 7, dadurch gekennzeichnet, daß nach Zugabe der Aggregationslösung und dem Inkubieren die Mischung bei ca. 1000 l/min zentrifugiert wird.

9. Verfahren nach einem der Ansprüche 1 bis 8, dadurch gekennzeichnet, daß die Mischung nach dem Neutralisieren mit 1000 1/min anzentrifugiert, geschüttelt und auf Agglutination geprüft wird.

10. Verfahren nach einem der Ansprüche 1 bis 9, dadurch gekennzeichnet, daß für einen Kontrollnachweis statt säurelöslicher Proteine aus menschlichen Leukozytenkernen eine Protaminlösung, z. B. aus Protamin-HCl verwendet wird.

11. Verfahren nach Anspruch 10, dadurch gekennzeichnet, daß für die Protaminlösung eine Stammlösung aus 1 g Protamin-HCl in 100 ml Aqua dest. hergestellt und diese im Verhältnis 1:10 mit 0,9 % NaCl verdünnt wird.

## Claims

1. Process for the detection of antibodies against erythocyte-specific antigens contained in the blond serum, in that a serum dilution is prepared with an erythrocyte suspension in a 0.9% NaCl solution as the antigen carrier and is mixed with a low ionic medium (LISS = low ionic strength solution), the mixture is incubated and then an aggregation solution in the form of acid-soluble proteins from human leucocyte nuclei is added, the mixture is incubated again and then centrifuged, the supernatant product is separated and the remaining mixture is neutralized with heparin-sodium or an organic acid and checked for agglutination.

2. Process according to claim 1, characterized in that a 2 to 5% erythrocyte suspension in a 0.9% NaCl solution is used as the antigen carrier.

3. Process according to claims 1 or 2, characterized in that for the aggregation solution is prepared a stock solution of 1 g of acid-soluble proteins from human leucocyte nuclei in 100 ml of distilled water and is diluted in a ratio of 1:10 with 0.9% NaCl to a working solution.

4. Process according to one of the claims 1 to 3, characterized in that for the neutralization solution a stock solution is prepared from 10 g of organic acid and 100 ml of distilled water and diluted in a ratio of 1:10 with 0.9% NaCl to a working solution.

5. Process according to claim 4, characterized in that citric acid or lactic acid is used as the organic acid.

6. Process according to one of the claims 1 to 3, characterized in that heparin-sodium pH 5, 5000 IU/ml is used for the neutralization solution.

7. Process according to one of the claims 1 to 6, characterized in that the mixture is incubated at ambient temperature.

8. Process according to one of the claims 1 to 7, characterized in that after adding the aggregation solution and incubation the mixture is centrifuged at approximately 1000 l/min.

9. Process according to one of the claims 1 to 8, characterized in that, after neutralization, the mixture is precentrifuged with 1000 l/min, shaken and checked for agglutination.

10. Process according to one of the claims 1 to 9, characterized in that for a controlled detection use is made of a protamine solution, e.g. from protamine-HCl, in place of acid-soluble proteins from human leucocyte nuclei.

11. Process according to claim 10, characterized in that for the protamine solution a stock solution is prepared from 1 g of protamine-HCl in 100 ml of distilled water and is diluted in a ratio of 1:10 with 0.9% NaCl.

**Revendications**

1. Procédé de détermination d'anticorps contenus dans le sérum sanguin contre des antigènes spécifiques pour érythrocytes, dans lequel une dilution de sérum est mélangée à une suspension d'érythrocytes dans une solution de NaCl à 0,9 % servant de porteur d'antigène et à un Low Ionic Medium (LISS = Low Ionic Strength Solution), le mélange étant incubé, une solution d'agrégation étant ensuite ajoutée sous forme de protéines solubles dans un acide et composées de noyaux de leucocytes humains, le mélange étant de nouveau incubé puis centrifugé, la partie supérieure étant séparée et le reste du mélange neutralisé à l'aide de sodium-héparine ou à l'aide d'un acide organique et soumis à un examen d'agglutination.

2. Procédé selon la revendication 1, caractérisé en ce que l'on utilise comme antigène une suspension d'érythrocytes de 2 à 5 % dans une solution de NaCl à 0,9 %.

3. Procédé selon la revendication 1 ou 2, caractérisé en ce que, pour la solution d'agrégation, on prépare une solution mère à partir de 1 g de protéines solubles dans un acide provenant de noyaux de leucocytes humains, dans 100 ml d'eau distillée, et on la dilue en une proportion de 1:10 avec NaCl à 0,9 % pour donner une solution utilisable.

4. Procédé selon l'une des revendications 1 à 3, caractérisé en ce que, pour la solution de neutralisation, on prépare une solution mère à partir de 10 g d'acide organique et 100 ml d'eau distillée et on la dilue en une proportion de 1:10 avec NaCl à 0,9 % pour donner une solution utilisable.

5. Procédé selon la revendication 4, caractérisé en ce que l'on utilise comme acide organique de l'acide citrique ou de l'acide lactique.

6. Procédé selon l'une des revendications 1 à 3, caractérisé en ce que l'on utilise pour la solution de neutralisation de l'héparine-sodium de pH 5, 5000 IU/ml.

7. Procédé selon l'une des revendications 1 à 6, caractérisé en ce que le mélange est incubé à la température ambiante.

8. Procédé selon l'une des revendications 1 à 7, caractérisé en ce qu'après adjonction de la solution d'agrégation et incubation, le mélange est centrifugé à à peu près 1000 l/min.

9. Procédé selon l'une des revendications 1 à 8, caractérisé en ce qu'après neutralisation, le mélange est concentré par centrifugation avec 1000 l/min, secoué et soumis à un examen d'agglutination.

10. Procédé selon l'une des revendications 1 à 9, caractérisé en ce que, pour effectuer une détermination de contrôle, au lieu de protéine soluble dans un acide et provenant de noyaux de leucocytes humains, on utilise une solution de protamine, par exemple de protamine-HCl.

11. Procédé selon la revendications 10, caractérisé en ce que, pour la solution de protamine, on prépare une solution mère à partir de 1 g de protamine-HCl dans 100 ml d'eau distillée et on la dilue en une proportion de 1:10 avec NaCl à 0,9 %.